(19)

Europäisches
Patentamt

European
Patent Office

Office européen
des brevets

(11) **EP 3 185 205 A1**

(12) **EUROPEAN PATENT APPLICATION**

(43) Date of publication:
**28.06.2017 Bulletin 2017/26**

(51) Int Cl.:
**G06Q 50/22** *(2012.01)*     **G06F 19/00** *(2011.01)*

(21) Application number: **15307081.8**

(22) Date of filing: **21.12.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Thomson Licensing
92130 Issy-les-Moulineaux (FR)**

(72) Inventors:
• **SCHMOUKER, Philippe
35576 Cesson-Sévigné (FR)**

• **HOWSON, Christopher
35576 Cesson-Sévigné (FR)**
• **DEFRANCE, Serge
35576 Cesson-Sévigné (FR)**
• **GAUTIER, Eric
35576 Cesson-Sévigné (FR)**
• **GUEGAN, Marie
35576 Cesson-Sévigné (FR)**

(74) Representative: **Huchet, Anne
TECHNICOLOR
1-5, rue Jeanne d'Arc
92130 Issy-les-Moulineaux (FR)**

(54) **METHOD AND DEVICE FOR DETECTING BEHAVIORAL PATTERNS OF A USER**

(57)    A system and method are provided for detecting a behavior patterns of a user. In one embodiment, the method comprises receiving information about activities of a user and calculating duration of each activity. Each activity that exceeds a threshold duration is categorized and labelled accordingly. Activities with similar labels are grouped and labelled activities are analyzing. Alerts are then generated for certain behavioral patterns such as those exhibiting anomalies.

**Figure 3**

| Initialization Step – Build a user profile and establish monitoring communication | 300 |

| Step 1  a) Log  activities – sensor/actuation (motion detector )/ key (remote etc.) – time in/out<br>        b) categorize into groups<br>            c) check for errors and remove it | 310 |

| STEP 2  - a) Order and group – chronologically by day/activity etc.<br>         b) split and rearrange items accordingly<br>         c) start/end time adjusted accordingly | 320 |

| STEP 3 – calculate duration of each activity/category | 330 |

| STEP 4 – establish threshold and arrange items based on that<br>        remove items that do not meet threshold – replace as unlabel data item | 340 |

| STEP 5 – replace unlabeled data item by content from adjacent neighbor;<br>        adjust duration time | 350 |

| STEP 6 – consider data items for same time of day that meet specific threshold;<br>        relabel those that do not reach new threshold as unlabeled data and combine with neighbor;<br>        analyze intersection of activities | 360 |

| STEP 7 – reassign unlabeled data from step 6 to neighbor and check again;<br>        repeat  steps 3 to 7 and set counter<br>        provide analysis/last processing and continually update | 370 |

EP 3 185 205 A1

**Description**

**TECHNICAL FIELD**

The present principles generally relates to a behavioral monitoring system and more particularly to an automated behavioral monitoring system that generates alerts based on previously recognized behavioral patterns.

**BACKGROUND**

This section is intended to introduce the reader to various aspects of art and to facilitate a better understanding of the various embodiments presented. Accordingly, it should be understood that these statements are to be read in this light, and not as admissions of prior art.

Development of technology and science has enabled the detection and monitoring of behavioral habits of individuals. There may be many reasons that detection and monitoring may be desirous such as prevention of criminal activity, addiction control and providing security to minors. However, a more popular use of such devices is to provide for the care of elderly, sick and disabled individuals. Care givers and medical professionals can diagnose problems and provide preemptive critical care by understanding behavioral patterns of these sick, elderly or handicapped individuals. Therefore understanding life patterns of these individuals can be used as a tool for diagnosis of behavioral or health problems. Anomalies can be detected for further investigation and alerts can be generated ahead of time and prior to occurrence of critical conditions to ensure timely action, especially in case of an emergency. In a different context, monitoring devices may be used for purposes of teaching. A patient with memory problems, for example can be reminded to turn off a stove after use or a blind person may receive assistance for navigating a particular room.

Unfortunately, current monitoring systems often rely exclusively on one type of input for generating alerts. The warning systems are also limited in the output they provide. For examples, conventional systems use motion sensing systems or sensors to detect movement. These motion sensors are typically photo-sensors that detect moving objects based on a variety of factors such as physical approximations of space or time. However, motion sensors are not discriminate and are often triggered by any motion. In other words, these systems cannot distinguish between different individuals or even other living beings such as pets. In addition, these systems are not sufficiently sophisticated to recognize the overall behavior patterns of individuals as they are only limited to the activities that occur within the footprint they monitor. Furthermore, because sensors are connected to an alarm circuit with an audible system, their use may be limited. For example, a deaf person or a person with severe hard of hearing may not be able to use this type of device as the only warning system involves audible sounds of a certain decibel.

Consequently, improved and reliable monitoring and alerting techniques are needed that can provide guidance and warnings to individuals discriminately. It would be more desirous if these techniques can provide an automated way to predict future potential issues prior to their occurrence.

**SUMMARY**

A system and method are provided for detecting a behavior patterns of a user. In one embodiment, the method comprises receiving information about activities of a user and calculating duration of each activity. Each activity that exceeds a threshold duration is categorized and labelled accordingly. Activities with similar labels are grouped and labelled activities are analyzed y. Alerts are then generated for certain behavioral patterns such as those exhibiting anomalies.

**BRIEF DESCRIPTION OF THE DRAWINGS**

The invention will be better understood and illustrated by means of the following embodiment and execution examples, in no way limitative, with reference to the appended figures on which:

Figure 1 depicts a block diagram for a data collection system according to an embodiment;

Figure 2 depicts a flow diagram for establishing a data base according to one embodiment; and

Figure 3 is a flow diagram of a multi-temporal habit detection system according to one embodiment.

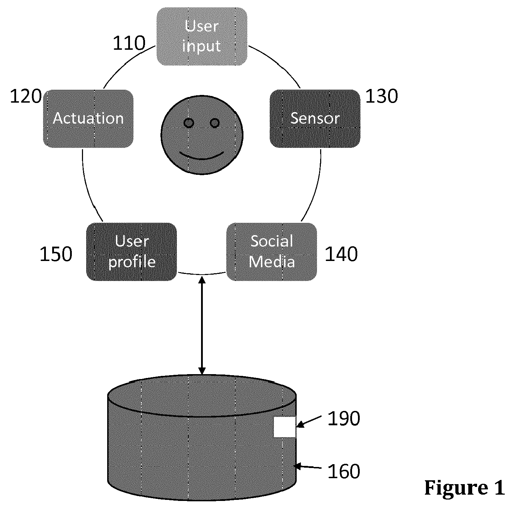

In Figure 1, the represented blocks are purely functional entities, which do not necessarily correspond to physically separate entities. Namely, they could be developed in the form of software, hardware, or be implemented in one or several integrated circuits, comprising one or more processors.

Wherever possible, the same reference numerals will be used throughout the figures to refer to the same or like parts.

## DESCRIPTION

It is to be understood that the figures and descriptions of the present principles have been simplified to illustrate elements that are relevant for a clear understanding of the present invention, while eliminating, for purposes of clarity, many other elements found in typical digital multimedia content delivery methods and systems. However, because such elements are well known in the art, a detailed discussion of such elements is not provided herein. The disclosure herein is directed to all such variations and modification

Understanding a behavior pattern can help in a variety of different settings. In elderly, understanding a particular behavior can help diagnose a problem like Alzheimer's and diabetes. Early detection can lead to better prognosis and can even prevent problems that are unrelated directly to the patient's conditions such as falls and other incidences that nevertheless cause injuries. Elderly, however, are not the only group that benefits by behavioral analysis. Warnings and alerts of a particular behavior may benefit a number of individuals such as minor children, addicts, those who may become victims of crimes and those who are incarcerated.

Patterns of behavior can be established over time in a number of ways. Figure 1, is an example of some of the ways where information about behavior patterns can be collected. The examples provided in Figure 1 only address a few ways of collecting information, with the understanding that many other methods are available as can be appreciated by those skilled in the art. In the example used in Figure 1, the few methods shown include gathering of information through user input 110, actuation 120, sensors 130, and on-line resources such as social media 140. Information may already exist and previously collected and stored in a user profile 150. To aid understanding, some of these methods can be explored in more detail.

As shown by numerals 110, information can be provided directly through a user directly through a user interface such as a computer keyboard, a touch screen or a mouse. The information can be about the user that is inputting the information or it may be about other individuals or even animals. For example, a care giver, a relative or a friend may provide information about a patient. Alternatively, a zoo keeper can input information about a particular animal such as a monkey to understand and automate behavioral patterns.

In one embodiment, the information is accumulated or transferred into the user profile or is directly logged such as by use or a computer or processor 190. The user profile may also reside in a storage location accessible by the computer or processor 190. In on embodiment, the user profile 150 may also include other type of information about the user or a third party. The user profile may include more than a single entry and already contain data that has been previously collected or that contain relevant information that is more inclusive than just the behavioral data. In the example above, the user profile of the monkey may include biological information such as the species and familiar relationship that may be important later on to the particular behavior.

In one embodiment, the computer or processor 190 may be part of a network or in processing communication with a network 160 of other devices including other computers and servers or through the Internet. Mobile and wireless devices, storage devices, displays and printers and other such components as can be appreciated by those skilled in the art can be part of this network. In addition, these computers can belong to the user/patient, care providers, patient relatives, hospital networks and others.

Besides direct input of information, behavioral patterns may be obtained through a number of ways. In the example provided in Figure 1 actuation and sensors are provided with the understanding that as can be appreciated by those skilled in the art, a number of other ways are available and can be used to obtain such data. For example, while a global positioning system is not shown, a GPS can also be used to gather movement of an individual or living being in a certain area. In addition, the sensor shown by reference numerals 120 may be representative of one or more sensors and can register a variety of functions and activities. For example, a temperature sensor can register the shifts of air or body temperatures, a light sensor can register every time a light switch is turned on or a motion sensor can monitor entering or a person to a particular room. Other similar sensors can be used to gather other information (e;g. ones with bluetooth or RF capabilities). In other embodiments, an actuator may be used instead of a sensor. For example, the actuator may be used every time the garage door is opened or in other similar manners. All such data are properly logged and processed such as by a processor or computer as shown by numerals 190 as discussed. The processor 190 can be in processing communication with other computers in a network as shown by that include storage locations, printers, displays and other such devices as can be appreciated by a person skilled in the art.

In addition, information may be collected through the use of on-line tools such as social media. For example, medical data may be gathered for a patient by accessing a variety of resources such as hospital databases, pharmacies and insurance companies. Social media that include occupation, blogs and other such information can also be accessed to add to this information.

One benefit of detecting habits in behaviors is to help make recommendation as well as providing alert settings.

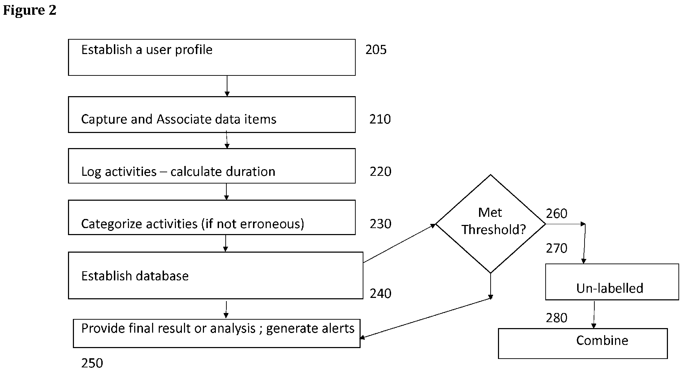

Recommenders will take benefit of known habits, while alert setters will take benefit of the detection of anomalous behaviors when compared to habits or alternatively because of them. In one embodiment, behavioral habits are evaluated over a particular period of time. In this way, if they are changing from a first set to a second set over time, the change itself is detected. In Figure 2, a flowchart depiction of one embodiment is illustrated that performs such a task. The data item is captured and logged as shown by numerals 210. A usage profile may exist or in one embodiment is established. If such is the case, the first step of the embodiment of Figure 2 would be the establishment of a user profile as shown in step 205. In either case, any user activity will be logged as shown in 220 of Figure 2 by means such as those already discussed in Figure 1.

In one embodiment, as shown in Figure 2, behavioral data item that is captured such as by ways discussed in Figure 1 may also include certain characterization as shown by numeral 230. In one example, these characterization features may include a starting timestamp (date, exact time etc.), an ending timestamp and a descriptive label. These characterization features can be preselected and customized depending on the type of activity that needs to be monitored. For example the time stamp may be intentionally designed to only capture a particular day or it may be more exacting and register not only the hour and minute in the day but also the second and so on. In another example the descriptive label may relate to a type of movie viewed, presence in one's home, occurrence of channel zapping, type of activity performed or any other type of category of things that are to be monitored or is of special interest. Additional processing may also occur. For example when time stamps are used, duration of the activity is calculated as shown in Figure 2.

In one embodiment, once the behavior data items are captured, they can then be associated each to one or more identified users or groups of users. These associations can be through establishment of a particular user profile (if not already in existence) or through other methods such as through a user ID or a set, list or group that are related to user(s). Each item may constitute an already identified set of behavioral data item or a new one.

When a user ID or user profile is used, one or more identifying devices may already be identified to be associated with the particular user. For example, in a patient, an emergency ID tag worn by the user and associated with a sensor may immediately mean that the data item has to be associated with a particular user. Other devices or a group of devices or a location of an activity, such as a user's home or bedroom, may also be tagged to immediately help associate a behavior with a user.

A database can then be established based on logged activities as shown by numerals 240. The database will be then analyzed over time (numeral 250 in Figure 2) to detect and identify behavioral data items associated with one or more specified users (subjects) or groups of users (subjects). These users/subjects are the individuals or other living things for which behavioral habits needs to be extracted over time.

Human, or even animal behaviors, are noisy by nature. Therefore, there are instances where a displayed activity is not representative of a particular habit but had occurred due to a particular non characteristic stimulus. Other activity may be erroneously logged or may be originated by a device associated with a user when in reality the activity was never originated by that user. Therefore, in one embodiment the erroneous entries such as these will be removed. In another embodiment, a threshold is established for the frequency of a certain activity to establish a habit. For example if the habit is to be evaluated over daily behaviors but for instance, the user does not go to bed or wake-up every day at the same time, these type of variations can be analyzed and either adjusted appropriately or eliminated from the analysis based on the threshold value. This is shown in numbers 260 through 280 and discussed in more detail later.

In one embodiment, the result of analyzing the results may require generating an alert. The alert may be generated when the analysis recognizes behavior patterns that exhibit anomalies. In an alternate embodiment, the alert may be used when the behavioral pattern matches a preselected behavior pattern such as from a list. The list may be provided such as in a storage location in the network 160 or be provided by accessing other databases or online resources. In on embodiment, the alert is sent to the user while in alternate embodiment the alerts can be sent to other individuals or other individuals and the user concurrently. For example, the user may be a comatose patent and therefore it will not be of much use to send the alert to the user. Instead, the alert will be provided immediately to a physician. In other embodiments, the alert can be sent to ambulances, patient's close family or care providers or others. In one embodiment, the type of alert and the information about the user will determine where the generated alert will be sent. In one embodiment, the network 160 will be used to provide the alert.

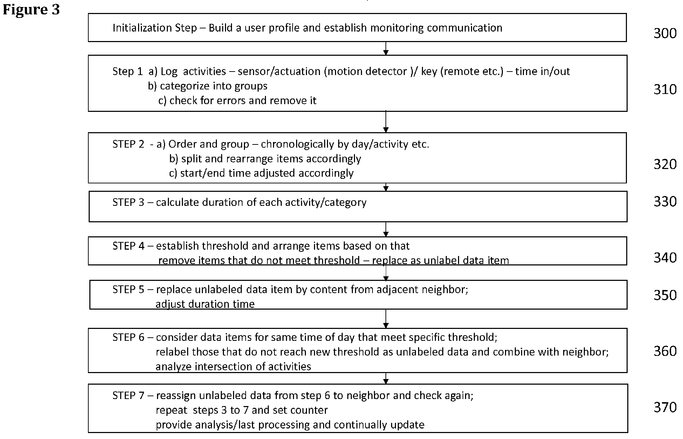

Figure 3 provides a flowchart depiction of one embodiment of the present invention. In a first step as shown by numerals 310, data items that are logged are associated with a user. As discussed in Figure 2, these can be timed and labelled. As discussed previously, in alternate embodiments, these can be logged based on direct user input, recordings, sensor identification (motion, door actuation, remote control key actuation etc.) or any other such means as can be appreciated by those skilled in the art. In one embodiment, logged data is then cleared of noise or other anomalous content (erroneous values or such) as discussed in conjunction with the embodiment as discussed in Figure 2.

In a second step as shown in Figure 3 by numerals 320, data items are then ordered. A number of different types of ordering can be used selectively. For example, the data items can be chronologically ordered, ordered by user groups, ordered by type of activity or other such manners. In one embodiment, the ordering can also have sub groups. For example, if the data is grouped chronologically, the grouping can be by days, either from midnight to next midnight or from noon to next noon, according to the targeted application. A resulting database table can then be generated in this example that contains days as entries and an ordered list of data items for every day. In some instances, this second step may lead to the splitting of some items over borders of time (the border being either midnight or noon according to the targeted application in some embodiments). In such cases, the starting and ending timestamps or other selection items may then have to be modified accordingly for these data items.

In a third step as shown by numeral 330, the duration of each data item is evaluated within each selected category. In the example used above where the date was of importance this may result in something such as:

$$duration = ending\ timestamp - starting\ timestamp$$

Referring back to Figure 3, in a fourth step as shown by numeral 340, a preselection category can then be used to separate data of a particular nature. This can be, for example, the threshold concept discussed in Figure 2. Referring back to Figure 2, the threshold 260 can be in on example a duration. For example, for every data item for which duration is less than a threshold, those items are removed from the data set and replaced by a corresponding "unlabeled" data item (numeral 270). Some strategies for the evaluation of the threshold are described in more detail subsequently.

In step 350, and as previously discussed in Figure 2 in 270, the unlabeled data items can be replaced by content from its timely preceding and following neighbors (in the example that uses time). In one embodiment, this can lead to the modification of the ending timestamp of the previous neighbor in time and of the starting timestamp of the next neighbor in time.

In step 360, data items are then considered for the same time of day (in the example that uses time) but over a particular ($k$) successive days. Different strategies for the definition of the number of these successive days can be used based on selection of categories and the intent of the database generation and analysis. In one embodiment, a specific intersection operation is processed on any period of time over these successive days: it is the max period of time for which all labels are the same over the successive days. For each of these $k$ days, any period of time that is not included in this intersection is labeled as "unlabeled" data item.

In step 370, the unlabeled data item (over days) can then be adjusted so that it is grouped together with its nearest or most closely related neighbor under its neighbor's label as appropriate. Steps 330 to 370 can also be repeated as many times as needed, but typical repetitions counts will range from 0 (no repetition) to 1. Once done, the description of the nearest previous day from "today" contains the most recent known behaviors habits over time per day. As an extension, deviations from habits may be evaluated a posteriori, in which case steps 6 and 7 are applied on neighbors days - i.e. previous and next ones - rather than only on the previous ones. This allows easy detection of any deviations of current ("today") behavior when compared to the evaluated last habits.

In one embodiment, the system and method provide alerts and warnings immediately or at the end of a particular period or both. To aid understanding, an example can be used in connection with the embodiments discussed in Figures 1 through 3. As can be appreciated, other embodiments can be provided in alternate embodiments associated with other examples and dependent on other uses. In this example a plurality of sensors such as motion and door sensors are associated with a user to provide actuation data to a connected box at home. The actuation data are to be sent to a processor such as one associated or connected to back-end of a server. The actuation data is collected within a database (DB) accessible by the processor.

The processing steps 300 to 370 as discussed in conjunction with the embodiment of Figure 3, can now be explored in more details and with the use of different examples to aid understanding. It is understood, however, that as can be appreciated by those skilled in the art, the examples that follow are only to aid understanding and other examples can be used in alternate embodiments.

Referring back now to step 310 of Figure 3, a system and method can be provided where for example the processor, is enabled to function as a Data Cleaner. In other words, the processor can remove anomalous data (e.g. too long duration or very rare duration, to be considered for instance as hardware or transmission failures). The processor can also detect anomalies and errors on a periodical basis. In one embodiment, the processor also acts as a Habits Evaluator. In other words, there is a periodical evaluation of the database. This means that database access leads to continual if not periodical evaluation of habits for each user(s) according to the other steps as discussed in Figure 3.

In one embodiment, the system and method provide alerts and warnings immediately or at the end of a particular period or both as discussed earlier. Therefore, the Habits Evaluator, can either immediately provide warnings on review of the DB or generate a report or recommendations (including warnings and alerts) at the end of a period.

In one embodiment, the results of a last day evaluation can be separately provided to a recommendation component (processor) to be incorporated as part of a recommendation report or to generate an alert with an urgency (immediate or part of the report). The data extracted by this Habits Evaluator, are then made available such as for example to front-end applications to provide recommendations (directly using evaluated habits), or to generate other alerts (based on evaluating deviation from evaluated habits).

In the example used, the behavioral data items can be associated with the room-presence of people in homes. For each home, these behavioral data items can be described and labelled in a particular way, as shown below:

- A label (e.g. living room, bedroom, kitchen, outside, ...)
- A starting timestamp (e.g. 2015-08-11 14:12:16.923+02:00)
- An ending timestamp (e.g. 2015-08-11 14:12:59.990+02:00)

Referring back to Figure 3, for this particular example, the first step 310 includes the activities that are timed and labelled behavioral accordingly (hereinafter references as data items). The logged information can be then stored or recorded and associated to one or more identified users. Such items can be either the direct recording of some sensors (e.g. motion sensor actuation, door sensor actuation, remote control keys actuation, etc.) or the result of some processing on sensors actuations samples (e.g. room presence at home) as discussed. This or a preliminary step can also be incorporated to remove noise or other anomalous content (erroneous values for sensors, for instance).

Applying this example to second step (320) in Figure 3, the data items are then chronologically ordered and grouped by days, either from midnight to next midnight or from noon to next noon, according to the targeted application. A resulting data table can be then generated. The table contains days as entries and an ordered list of data items for every day. As discussed previously, this may trigger the need to split some items over borders of time (the border being either midnight or noon according to the targeted application. Starting and ending timestamps are then modified accordingly for these data items. For instance, a representation of such a dataset can be (JSON form):

```
{
        "2015-08-11": [
       {"label": "bedroom",
        "start": "2015-08-11 00:00:00.000+02:00",
        "end" : "2015-08-11 07:11:24.359+02:00" },
       {"label": "bathroom",
        "start": "2015-08-11 23:59:25.010+02:00",
        "end" : "2015-08-11 23:59:59.999+02:00"}
       ],
       "2015-08-12":
       {"label": "bathroom",
        "start": "2015-08-12 00:00:00.000+02:00",
        "end" : "2015-08-12 00:00:56.789+02.00"},
       ],
   }
```

Applying the third step of Figure 3, the duration of each data can then be evaluated within each day in this example using *duration = ending timestamp-starting timestamp.* This step can in some ways be thought of as the continuation of the previous one but that is separated from it here for descriptive purpose. For example, in one embodiment, the data set can now represent:

```
{
        "2015-08-11":
       {"label": "bedroom",
        "start": "2015-08-11 00:00:00.000+02:00",
        "end" : "2015-08-11 07:11:24.359+02:00",
             "duration": "07:11:24.359"},
       {"label": "bathroom",
        "start": "2015-08-11 23:59:25.010+02:00",
        "end" : "2015-08-11 23:59:59.999+02:00",
             "duration": "00:00:34.989"}
       ],
       "2015-08-12": [
       {"label": "bathroom",
        "start": "2015-08-12 00:00:00.000+02:00",
        "end" : "2015-08-12 00:00:56.789+02.00",
             "duration": "00:00:56.789"},
       ],
   }
```

Where durations could as well be float values representing seconds as well:

```
{
        "2015-08-11": [
     {"label": "bedroom",
      "start": "2015-08-11 00:00:00.000+02:00",
      "end" : "2015-08-11 07:11:24.359+02:00",
              "duration": 25884.359},
      {"label": "bathroom",
      "start": "2015-08-11 23:59:25.010+02:00",
      "end" : "2015-08-11 23:59:59.999+02:00",
              "duration": 34.989}
     ],
     "2015-08-12": [
      {"label": "bathroom",
      "start": "2015-08-12 00:00:00.000+02:00",
      "end" : "2015-08-12 00:00:56.789+02.00",
              "duration": 56.789},
     ],
}
```

If some type of threshold is now applied as suggested in 340 in Figure 3, then every item for which duration was less than the present threshold will be removed. In that case the data set that is removed will be replaced by corresponding unlabeled data items.

In the example shown, to aid understanding, a 53 days period is chosen for explanatory purposes. In one embodiment, a particular algorithm is used as follows :

> *for every labelled period of time:*
>
> > *shorten it by threshold/2 seconds on both of its starting and ending sides.*

In this way, the threshold value can be set in many ways. Some examples include: 1) by providing a fixed value (e.g. from few seconds to few minutes); and 2) by evaluating the repartition law of the labeled durations and setting the threshold in one of these ways. The repartition law can be comprised of a list itself. Some items that may be on this list may include: being the max value of the n shorter ones - n to be arbitrarily chosen or evaluated according to some algorithm; being the max value of the p % shorter ones - p to be arbitrarily chosen or evaluated according to some statistical algorithm; and after a clustering step on durations, as being the min value over all clusters of the max value within each cluster.

Progressing to step 350, every unlabeled data item can be replaced by its neighbor's content. In one embodiment, this will in turn leads to the modification of the ending timestamp of the previous neighbor in time and of the starting timestamp of the next neighbor in time. In this respect, many algorithms can be used to replace unlabeled content per labeled one. The one we propose is this one:

> *for every day:*
>
> > *for every unlabeled period of time:*
> >
> > > *add half of its duration to previous labeled period of time*
> > >
> > > *add half of its duration to next labeled period of time*

In one embodiment, this may lead to some special cases. In this example, these cases may be the possible three situations:

> *1. if there is no previous labeled period of time:*
>
> > *add all unlabeled period duration to next labeled period of time*
>
> *2. if there is no next labeled period of time:*

*add all unlabeled period duration of previous labeled period of time*

3. *if these are neither previous nor next labeled periods of time:*

*set the whole day duration as an unlabeled period*

Referring now to the next processing stage as discussed in conjunction with 360 in Figure 3 and applying this example, data items can now be considered for the same period of time of day but over *k* successive days.

A specific intersection operation is processed on same period of time over these k successive days: it is the max period of time for which all labels are the same over the successive days. For each of these days, any period of time that is not included in this intersection is labeled as "unlabeled" data item.

The results can be seen in the next stage where , the value of *k* is chosen to equal to 3. Here, Two kinds of algorithms can be used to remove labeled periods of times over days. One of them deals with the a posteriori evaluation of habits while the other type of algorithm deals with the evaluation of the most recent habits.

Type 1 - a posteriori evaluation of habits for day *day*
*evaluate intersection of time periods from day-k/2, to day+k/2*
Type 2 - evaluation of the most recent habits for day *day*
*evaluate intersection of time periods from day-k to day-1*

A description of the evaluation of the intersection of the labeled time periods over the k-length period of days is provided in one example as provided below.

```
// let's create a temporary list of unlabeled periods of times
    tmp_list := empty_list
    for each day in the list of the k successive days:
       for each labeled period in day:
           instantiate new data_item
           data_item. starting timestamp := period. starting_timestamp
           append data_item to tmp_list
    // let's sort them in chronological order and evaluate missing attributes
    sort tmp_list on starting_timestamps values
    for each period in tmp_list but the last one:
       period.ending_timestamp := next_period.starting_timestamp
        tmp_list[last period].ending_timestamp := midnight
    // then, let's evaluate the intersection of labels over the temporary periods of time
    for each period in tmp_list:
        k_labels_set := empty _set
       for each day in the list of successive days:
           label := (find corresponding period within day).label
           add label to k-labels-set
        if card(k_labels_set) > 1:
           period.label := 'unlabeled' ll not all labels are the same
        else:
           period.label := k_labels_set.get_item(0)
    ll finally, modify the description of current day
    user_data_set [day]:= tmp_list
```

In this case, since each period of time within tmp_list is shorter than all corresponding periods of time in the k successive days, the way to "find corresponding period within (a specific) day" is straightforward:

*for each period_of_time in day:*

*if     period_of_time.starting_timestamp     $\leq$     searched_period.starting_timestamp     and searched_period.starting_timestamp $\leq$ period_of_time.ending_timestamp:*

*return period_of_time*

The number of days over which this processing is to be done, *k,* can either be arbitrarily set, according to the targeted applications; or be evaluated according to many types of algorithms (see subsection "Fourth Step"). Once set, this value k is used for Steps 6 and 7 of Figure 3 shown by numerals 360 and 370.

In step 370, as discussed every unlabeled data item over days gets its nearest older neighbor's label. As an extension, deviations from habits may be evaluated a posteriori, in which case steps 6 and 7 are applied on neighbors days - i.e. $k$/2 previous and $k$/2 next ones - rather than only on the $k$ previous ones.

In one embodiment, as discussed in Figure 3, steps 330 to 370 can then be repeated as many times as needed, but typical repetitions counts will range from 0 (no repetition) to 1. In this regard, when steps 4 and 5 (340 and 350) are being repeated or alternatively in other embodiments, the too short periods for time within every day can be removed and replaced by unlabeled periods of time with that of its neighborhoods. In addition for step 360,the nonuniform periods of time can also be removed over the successive days and instead be replaced by unlabeled periods of time with that neighborhoods data over successive days. Once done, the description of the nearest previous day from "today" contains the most recent known behaviors habits over time per day. It has to be understood that real processing would: 1) only be done on $k$ most recent days, with $k$ to be arbitrarily set, e.g. 30 days or 3 months according to the use case; or 2) provide as a result the only habits as evaluated for the day before 'today'

Meanwhile, having shown results over a longer period shows that, for every day - 1, evaluated habits may change over time which is exactly the targeted information to be extracted. As an extension, successive days used for the extraction of habits may not be the successive days in months. They could be:

- the same day of the week over successive weeks

  ○ every Monday,
  ○ every Tuesday,
  ○ ...

- the same group of days in the week:

  ○ weekday
  ○ weekend

- the successive days per season, over years:

  ○ all days of spring, over successive years
  ○ all days of summer, over successive years
  ○ ...

- The same day of the week over successive weeks pre season

**Claims**

1. A method for detecting behavioral patterns of a user, comprising:

   receiving (300) information about activities of a user;
   calculating (200) duration of each activity;
   categorizing (230) each activity that exceeds a threshold duration and labelling them accordingly;
   grouping (320) activities with similar labels ; and
   analyzing (250) labelled activities for behavioral patterns and generating alerts when behavior patterns exhibit anomalies.

2. The method of claim 1, wherein an alert (250) is issued when said behavioral patterns match a preselected behavior pattern.

3. The method of claim 2, wherein said preselected behavior patterns are obtained from a storage location (160).

4. The method of claim 1, further comprising: replacing outliers from a set of labeled activities with neighbors (350) labeled activities, wherein said neighbors are of closest labelled activity.

5. The method of claim 1, wherein information about a user activity and its duration is received from a sensor (130).

6. The method of claims 1-3 wherein information about a user activity is inputted using a user interface (110).

7. The method of claims 1-4, further comprising establishing a user profile such that information about the user is logged in the user profile (150).

8. The method of claims 1 or 7, wherein information is gathered about a plurality of users and each user behavior is analyzed discriminately and separately from other users (300).

9. The method of claims 1 or 8, wherein information about a user is collected from searching on-line resources (140).

10. The method of claims 1 or 9, wherein data about a user is analyzed and potential errors (310) are flagged and the flagged data is removed.

11. The method of claims 1 or 9, wherein data is analyzed continuously on receipt of new information.

12. The method of claims 1 or 11, wherein a report (250) can be generated about the user activity.

13. The method of claims 1 or 12, wherein the generated alert (250) is sent to the user.

14. The method of claims 1 or 13, wherein the generated alert (250) is sent to at least one person who is not the user.

15. A system for monitoring behavior patterns, comprising:

a processor (190) configured for monitoring a user activity;
a storage location (160) for logging user activity, said storage location also containing a user profile for each user having information about each of said user's behavior;
at least a sensor ('30) and a user interface (110) enabled to receive user activity information and communicate said information with said processor;
said processor logging said activity information in said user profile (150) and calculating duration of each activity;
said processor (190) analyzing said logged activity information and duration such that said activities are categorized accordingly based on their type and duration;
said processor generating alerts (250) based on said analysis.

Figure 1

**Figure 2**

| Establish a user profile | 205 |

↓

| Capture and Associate data items | 210 |

↓

| Log activities – calculate duration | 220 |

↓

| Categorize activities (if not erroneous) | 230 |

↓

| Establish database |

240

↓

| Provide final result or analysis ; generate alerts |

250

Met Threshold?  260

270

| Un-labelled |

280

| Combine |

**Figure 3**

| Initialization Step – Build a user profile and establish monitoring communication | 300 |

| Step 1  a) Log  activities – sensor/actuation (motion detector )/ key (remote etc.) – time in/out<br>          b) categorize into groups<br>               c) check for errors and remove it | 310 |

| STEP 2  - a) Order and group – chronologically by day/activity etc.<br>            b) split and rearrange items accordingly<br>            c) start/end time adjusted accordingly | 320 |

| STEP 3 – calculate duration of each activity/category | 330 |

| STEP 4 – establish threshold and arrange items based on that<br>         remove items that do not meet threshold – replace as unlabel data item | 340 |

| STEP 5 – replace unlabeled data item by content from adjacent neighbor;<br>         adjust duration time | 350 |

| STEP 6 – consider data items for same time of day that meet specific threshold;<br>         relabel those that do not reach new threshold as unlabeled data and combine with neighbor;<br>         analyze intersection of activities | 360 |

| STEP 7 – reassign unlabeled data from step 6 to neighbor and check again;<br>         repeat  steps 3 to 7 and set counter<br>         provide analysis/last processing and continually update | 370 |

Europäisches
Patentamt

European
Patent Office

Office européen
des brevets

**EUROPEAN SEARCH REPORT**

Application Number

EP 15 30 7081

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/131736 A1 (NELSON KYLE S [US] ET AL) 16 June 2005 (2005-06-16) <br> * abstract; figures 1,4, 5A,5B,7,8 * <br> * paragraphs [0006] - [0008] * <br> * paragraphs [0023] - [0033] * <br> * paragraphs [0046], [0047] * <br> * paragraphs [0052] - [0063], [0066] * <br> * paragraphs [0073] - [0086] * <br> ----- | 1-15 | INV. <br> G06Q50/22 <br> G06F19/00 |
| X | TRUONG T B T ET AL: "Alert management for home healthcare based on home automation analysis", <br> 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, <br> 31 August 2010 (2010-08-31), pages 2128-2131, XP032108986, <br> DOI: 10.1109/IEMBS.2010.5627249 <br> ISBN: 978-1-4244-4123-5 <br> * the whole document * <br> * page 2129 * <br> ----- | 1-15 | |
| X | WO 2015/127491 A1 (UNIV MONASH [AU]) 3 September 2015 (2015-09-03) <br> * abstract; figures 1,3,4,7 * <br> * paragraphs [0001], [0007] - [0010] * <br> * paragraphs [0029] - [0031] * <br> * paragraphs [0058] - [0064], [0068] - [0070] * <br> ----- <br> -/-- | 1-15 | TECHNICAL FIELDS SEARCHED (IPC) <br><br> G06Q <br> G06F |

The present search report has been drawn up for all claims

| Place of search | Date of completion of the search | Examiner |
|---|---|---|
| Munich | 27 May 2016 | Bauer, Rodolphe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

**EUROPEAN SEARCH REPORT**

Application Number

EP 15 30 7081

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ENAMUL HOQUE ET AL: "AALO: Activity recognition in smart homes using Active Learning in the presence of Overlapped activities", PERVASIVE COMPUTING TECHNOLOGIES FOR HEALTHCARE (PERVASIVEHEALTH), 2012 6TH INTERNATIONAL CONFERENCE ON, IEEE, 21 May 2012 (2012-05-21), pages 139-146, XP032201831, ISBN: 978-1-4673-1483-1 * abstract * * page 139; table I * * page 143 - page 145 * | 1-15 | |
| A | AKINORI FUJII ET AL: "Behavior description algorithm based on home sensor data using nonlinear transformations", NETWORKED SENSING SYSTEMS, 2008. INSS 2008. 5TH INTERNATIONAL CONFERENCE ON, IEEE, PISCATAWAY, NJ, USA, 17 June 2008 (2008-06-17), pages 63-66, XP031314963, ISBN: 978-4-907764-31-9 * the whole document * | 1-15 | TECHNICAL FIELDS SEARCHED (IPC) |

The present search report has been drawn up for all claims

| Place of search | Date of completion of the search | Examiner |
|---|---|---|
| Munich | 27 May 2016 | Bauer, Rodolphe |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 15 30 7081

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2016

| Patent document cited in search report | | Publication date | Patent family member(s) | | Publication date |
|---|---|---|---|---|---|
| US 2005131736 | A1 | 16-06-2005 | AT | 458236 T | 15-03-2010 |
| | | | AT | 520108 T | 15-08-2011 |
| | | | CA | 2551184 A1 | 07-07-2005 |
| | | | EP | 1700281 A2 | 13-09-2006 |
| | | | EP | 1870864 A1 | 26-12-2007 |
| | | | HK | 1096756 A1 | 02-12-2011 |
| | | | US | 2005131736 A1 | 16-06-2005 |
| | | | US | 2014074504 A1 | 13-03-2014 |
| | | | WO | 2005062267 A2 | 07-07-2005 |
| WO 2015127491 | A1 | 03-09-2015 | NONE | | |

EPO FORM P0459

For more details about this annex : see Official Journal of the European Patent Office, No. 12/82